# EUROPEAN PATENT APPLICATION

(11) **EP 4 278 958 A1**
(43) Date of publication of application: **22.11.2023**
(21) Application number: 22174242.2
(22) Date of filing: 19.05.2022
(51) Int. Cl.: A61B 5/0205, A61B 5/01, A61B 5/282, A61B 5/00, A61B 5/053

(54) **DEVICE FOR INTERFACING A USER WITH A PLURALITY OF MEDICAL DEVICES AND A SYSTEM COMPRISING SUCH DEVICE**

(71) Applicant: Capsula S.r.l., 20121 Milano (MI) (IT)
(72) Inventor: Andreoni, Giuseppe, 20835 Muggiò MB (IT); Nizardo Chailly, Alessandro, 20121 Milano MI (IT)
(74) Representative: Perani & Partners S.p.A.

(57) **Abstract**

Device (1) for interfacing a user with a plurality of medical devices (6, 6', 6") comprising: a plurality of sensors (2, 2', 2") each configured to detect a physical and/or physiological quantity related to a user so as to generate a respective detection signal; a panel (7) adapted to house at least the plurality of sensors (2, 2', 2") and configured to interface the plurality of sensors (2, 2', 2") with a user; a plurality of user protection devices (3, 3', 3"), each associated with a respective sensor of the plurality of sensors (2, 2', 2"); a plurality of filtering devices (4, 4', 4"), each associated with a respective sensor of the plurality of sensors (2, 2', 2") and configured to filter the respective detection signal so as to generate an output signal; a plurality of connection terminals (5, 5', 5") each connectable with a medical device (6, 6', 6") for transmitting the respective output signal.

## Description

### FIELD OF APPLICATION

The present invention relates to a device that allows to interface a user with a plurality of medical devices and a system comprising such device.

### Description of the prior art

It is well known in the art the Internet of Medical Things (IoMT, as abbreviated) approach, which allows to obtain integrated systems of medical devices capable of measuring a plurality of different physiological signals. Each physiological signal has its own acquisition chain, such as an electronic analogue sensor detecting the signal from the patient, a specific processing system for the signal detected by the sensor, a display system for the processed data and finally a data transmission system.

### Problem of the prior art

In such systems, there may be a need to replace a device for a given physiological signal, e.g. by replacing it with a device that is different in design/manufacturer but equivalent in characteristics and type of signal measured (e.g. a new electrocardiograph) in order to increase performance and/or safety, and/or miniaturisation. Such a replacement could easily prove to be feasible with regard to the software integration thanks to standard protocols such as, for instance, Bluetooth or appropriate software APIs. However, the replacement of the medical device also involves the structural aspect, which entails an expensive hardware redesign and possible recertification of the system with the new medical device. In fact, the new medical device that is to be integrated into the system has its own specific physical interface for the sensors coupled thereto. For example, if one wanted to replace a device for measuring a single electrocardiographic lead for assessing heart rhythm alterations or a stress state by means of heart rate variability (HRV) analysis, it would be necessary to redesign the physical interface of the sensors as it would be profoundly different in the new device to be integrated into the system.

### SUMMARY OF THE INVENTION

Object of the present invention is being able to replace and/or integrate medical devices in a standardised, easy and practical manner into an IoMT system capable of overcoming the drawbacks of the prior art.

The technical task outlined and the objects specified are substantially achieved by a device and related system comprising the technical features set forth in one or more of the appended claims.

### Advantages of the invention

Thanks to an embodiment, it is possible to obtain a device that allows to interface a user with a plurality of medical devices.

Thanks to an embodiment, it is possible to create a sensitive device wherein the medical devices that can be connected thereto can be easily replaced or integrated without making hardware changes to the device.

Thanks to an embodiment, it is possible to make a system comprising the device of the invention.

### BRIEF DESCRIPTION OF THE DRAWINGS

The characteristics and advantages of the present invention will become clear from the following detailed description of a possible practical embodiment, illustrated by way of a non-limiting example in the set of drawings, wherein:
- Figure 1 shows a schematic view of a device according to the present invention,
- Figure 2 shows a schematic view of a system comprising the system of Figure 1,

- Figure 3 shows a first embodiment of the system of Figure 2,
- Figure 4 shows a second embodiment of the system of Figure 2.

The device and the related system illustrated in the enclosed figures are to be understood as represented schematically, not necessarily to scale and not necessarily with the proportions of the various constituent elements represented.

### DETAILED DESCRIPTION

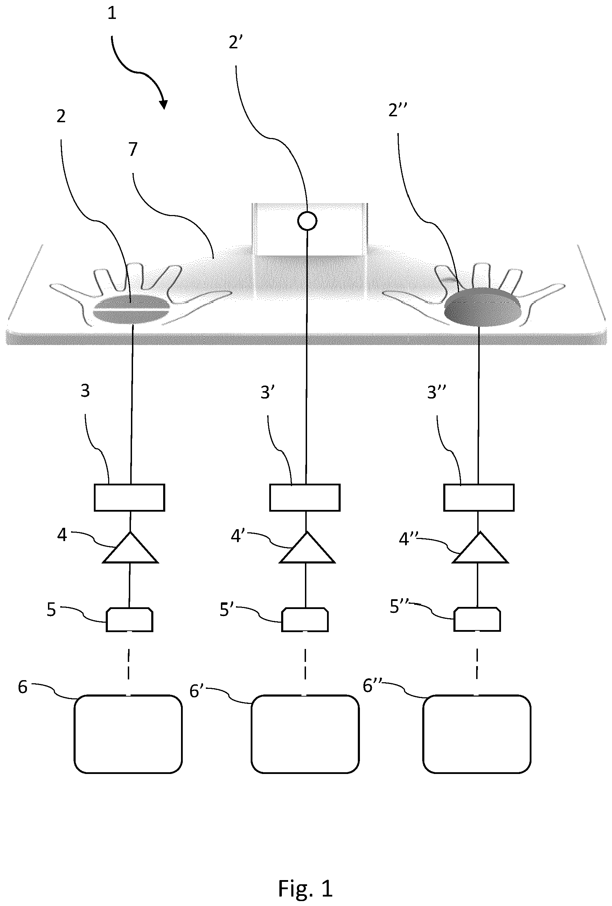

The present invention relates to a device 1, shown in Figure 1, which, in use, allows to interface a user with a plurality of medical devices 6, 6', 6" external to the device 1. The connection of the device 1 with medical devices advantageously allows to perform measurements of physiological user signals using a single interface.

The device 1 comprises a plurality of sensors 2, 2', 2", wherein each sensor is configured to detect a respective physical quantity related to a user so as to generate a corresponding detection signal. Preferably, the physical quantities detected by the sensors comprise one or more of biological, bioelectric and mechanical contact and/or thermal signals.

The device 1 comprises a panel 7 adapted to house at least the plurality of sensors 2, 2', 2". The panel 7 is shaped to interface the plurality of sensors 2, 2', 2" with one user. It should be emphasised that the panel 7 is a hardware element that not only supports and houses the sensors 2, 2', 2", but also allows a practical and secure interface of the same sensors with the user to detect physical quantities and simultaneously generate the detection signal. The panel 7 may have a variety of configurations depending on the type of sensors supported and their location on the panel, so that the sensors are accessible to the user to carry out the measurements.

The device 1 comprises a plurality of user protection devices 3, 3', 3", whereby each user protection device is associated with a respective sensor of the plurality of sensors 2, 2', 2" to ensure the protection of the patient from any electrostatic and electrical discharges due to failures or malfunctions. Preferably, user protection devices incorporate a grounding system for the patient's safety and additional protection against electrostatic discharge. In the exemplary case wherein sensors include ECG electrodes, the respective protection devices may be provided with ESD diodes to protect against electrical discharges concerning the patient in the event of failure of the ECG equipment connected to the device 1. Advantageously, there is a double protection of the patient since a basic protection against electric discharges is always present in the medical device connected to the device 1.

The device 1 comprises a plurality of filtering devices 4, 4', 4", wherein each filtering device is associated with a respective sensor of the plurality of sensors 2, 2', 2" and is configured to filter the respective detection signal in order to generate an output signal. It is worth noting that the output signal is a filtered signal, cleared from any background noise, so as to be compatible with the medical devices 6, 6', 6" that can be associated with the respective sensors 2, 2', 2" . Advantageously, the device 1 is electrically shielded to eliminate EMC induction artefacts such as fluctuations at the mains current frequency, which can also be further improved with the integration of ferrite filters.

The device 1 also comprises a plurality of connection terminals 5, 5', 5", each connectable with a medical device 6, 6', 6" for transmitting the respective output signal. It is worth noting that the connection terminals allow easy and convenient connection to medical devices, which may be easily replaced, disconnected and reconnected with the connection terminals 5, 5', 5". Such terminals have noiseshielded connections that allow the output signals to be transferred without undergoing alterations. Preferably, the connection terminals have a very low impedance, i.e. with a maximum resistance <50 ohms. Always preferably, the connection terminals comprise terminals with button connection, especially suitable for the connection in case the sensors comprise Ag/AgCl ECG electrodes with button connection. Alternatively, the connection terminals may be of the bayonet or multi-pin connector type. Always preferably, in the case of flat steel electrodes, it is possible to use the clamp electrode for the connection.

According to a preferred solution of the invention, the plurality of sensors 2, 2', 2" comprises a sensor for measuring the oxygen saturation in the user's blood.

According to a preferred embodiment, the plurality of sensors 2, 2', 2" comprises an infra-red temperature sensor.

According to a further preferred embodiment, the plurality of sensors 2, 2', 2" comprises a contact temperature sensor.

According to a preferred solution of the invention, the plurality of sensors 2, 2', 2" comprises a plurality of electrocardiogram (ECG, as abbreviated) electrodes.

Preferably, the plurality of sensors 2, 2', 2" comprises one or more electrodes for implementing a single lead for measuring the heart rate (or HR, as abbreviated) and its variability.

Still preferably and with reference to Figure 1, the electrodes may be a pair of flat sensors 2, more preferably each having a semi-circular shape. Alternatively, the electrodes 2" may be shaped as a pair of sphere slices, more preferably a pair of sphere quarters. The latter morphology of the sensors 2" has an ergonomic shape that eases laying the user's hand by accommodating the hollow of the palm.

According to a further preferred embodiment of the invention, the plurality of sensors 2, 2', 2" comprises a plurality of electrodes for electrodermal activity (EDA, as abbreviated).

Also according to a preferred embodiment, the plurality of sensors 2, 2', 2" comprises a plurality of bioimpedance electrodes.

According to a preferred embodiment of the invention, the plurality of sensors 2, 2', 2" comprises at least one optical sensor.

Advantageously, the device 1 of the invention allows for multi-signal and universal measurement, as it is compatible and connectable with any medical device suitable for measuring the signal detected by the corresponding sensor of the device 1.

Advantageously, the device 1 may be connected interchangeably with any medical device without having to make any structural changes to the device of the invention.

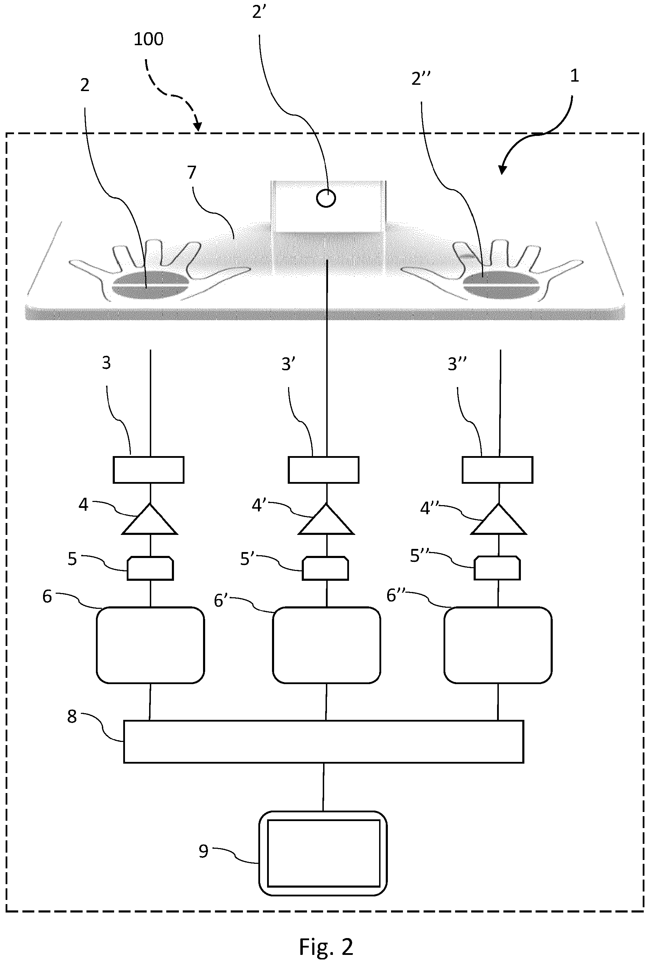

The present invention also relates to a system 100, schematically shown in Figure 2, comprising a device 1 according to the invention.

The system 100 comprises a plurality of medical devices 6, 6', 6", each connectable with a respective terminal of the plurality of connecting terminals 5, 5' 5" of the device 1. The medical devices 6, 6', 6" are configured to receive and process output signals in order to generate respective measurement signals. It should be noted that the medical devices may be disconnected and reconnected with the connection terminals for maintenance or quick replacement thereof.

The system 100 also comprises a processing unit 8 in signal communication with the plurality of medical devices 6, 6', 6" to receive and process measurement signals.

The system 100 also comprises a graphic interface 9 associated with the processing unit 8 for displaying information related to the measurement signals processed by the processing unit 8. Advantageously, the system 100 allows to generate and display reports of the results of the user's physiological parameters detected by the sensors 2, 2', 2".

In accordance with a preferred embodiment, the system 100 comprises a printer 10 associated with the processing unit 8 and configured to print information relating to the measurement signals processed by the processing unit 8 and displayed on the graphic interface 9.

Preferably, the system 100 comprises power supply means for electrically powering at least the medical devices 6, 6', 6", the processing unit 8 and the graphic interface 9.

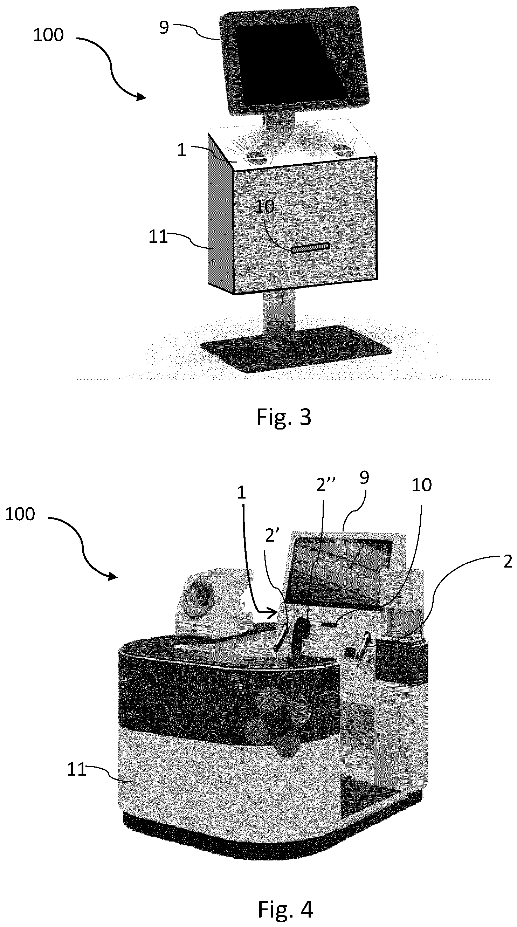

Preferably, the system 100 comprises a casing 11 adapted to house at least the device 1. With reference to Figure 3, the casing 11 is shaped like a totem allowing a convenient and comfortable interface between user and device 1, and in particular between user and sensors 2, 2', 2". In the example of Figure 3, the casing 11 also houses therein the processing unit and the medical devices, in addition to the printer 10 and the graphic interface 9

According to the embodiment of Figure 4, the casing 11 is made in the form of a workstation adapted to accommodate a user, being provided with an ergonomic seat allowing a comfortable and convenient interface between the user and the device 1, and especially between the user and the sensors 2, 2', 2".

## Claims

1. A device (1) for interfacing a user with a plurality of medical devices (6, 6', 6"), **characterised in that** it comprises:
- a plurality of sensors (2, 2', 2") each configured to detect a physical and/or physiological quantity related to a user so as to generate a respective detection signal;
- a panel (7) adapted to house at least the plurality of sensors (2, 2', 2") and shaped to interface the plurality of sensors (2, 2', 2") with a user;
- a plurality of user protection devices (3, 3', 3"), each associated with a respective sensor of the plurality of sensors (2, 2', 2");
- a plurality of filtering devices (4, 4', 4"), each associated with a respective sensor of the plurality of sensors (2, 2', 2") and configured to filter the respective detection signal in order to generate an output signal;
- a plurality of connection terminals (5, 5', 5") each connectable with a medical device (6, 6', 6") for transmitting the respective output signal.

2. The device (1) according to claim 1, wherein
- the plurality of sensors (2, 2', 2") comprises an SPO2 sensor.

3. The device (1) according to claim 1 or 2, wherein
- the plurality of sensors (2, 2', 2") comprises an infra-red temperature sensor.

4. The device (1) according to any one of the preceding claims, wherein
- the plurality of sensors (2, 2', 2") comprises a contact temperature sensor.

5. The device (1) according to any one of the preceding claims, wherein
- the plurality of sensors (2, 2', 2") comprises a plurality of electrocardiogram electrodes.

6. The device (1) according to any one of the preceding claims, wherein
- the plurality of sensors (2, 2', 2") comprises a plurality of electrodes, either flat or shaped like a sphere segment.

7. The device (1) according to any one of the preceding claims, wherein
- the plurality of sensors (2, 2', 2") comprises a plurality of electrodes for electrodermal activity.

8. The device (1) according to any one of the preceding claims, wherein
- the plurality of sensors (2, 2', 2") comprises a plurality of electrodes for bioimpedance testing.

9. The device (1) according to any one of the preceding claims, wherein
- the plurality of sensors (2, 2', 2") comprises at least one optical sensor.

10. A system (100) comprising:
- a device (1) according to any one of the preceding claims;
- a plurality of medical devices (6, 6', 6") connectable with the plurality of connection terminals (5, 5', 5") of the device (1) and configured to receive and process the output signals in order to generate respective measurement signals;
- a processing unit (8) in signal communication with the plurality of medical devices (6, 6', 6") to receive and process measurement signals;
- a graphic interface (9) associated with the processing unit (8) to display information about the measurement signals processed by the processing unit (8).

11. The system (100) according to claim 10, comprising:
- a printer (10) associated with the processing unit (8) and configured to print out information about the measurement signals processed by the processing unit (8) and displayed on the graphic interface (9).

12. The system (100) according to claim 10 or claim 11, comprising:
- a casing (11) capable of housing at least the device (1).

13. The system (100) according to claim 11, wherein
- the casing (11) is a totem or a location adapted to accommodate a user.
